# EUROPEAN PATENT SPECIFICATION

(11) **EP 2 491 104 B1**
(45) Date of publication and mention of the grant of the patent: **02.01.2019**
(21) Application number: 10825511.8
(22) Date of filing: 19.10.2010
(51) Int. Cl.: A61Q 5/02, A61Q 19/10, A61K 8/19, A61K 8/20, A61K 8/44, A61K 8/46, C11D 1/28, C11D 1/90, C11D 1/92, C11D 1/94, C11D 17/00

(54) **VISCOUS LIQUID CLEANSING COMPOSITIONS COMPRISING SULFONATED FATTY ACIDS, ESTERS, OR SALTS THEREOF AND BETAINES OR SULTAINES**
VISKÖSE FLÜSSIGKEITSREINIGUNGSZUSAMMENSETZUNGEN MIT SULFONIERTEN FETTSÄUREN, ESTERN ODER SALZEN DARAUS UND BETAINEN ODER SULTAINEN
COMPOSITIONS NETTOYANTES LIQUIDES VISQUEUSES COMPRENANT DES ACIDES GRAS SULFONÉS, DES ESTERS OU DES SELS DE CEUX-CI ET DES BÉTAÏNES OU DES SULTAÏNES

(30) Priority: 21.10.2009 US 253709 P
(43) Date of publication of application: 29.08.2012
(73) Proprietor: Stepan Company, Northfield, Illinois 60093 (US)
(72) Inventor: Dong, Xue Min, Lincolnshire Illinois 60069 (US); Sajic, Branko, Lincolnwood Illinois 60712 (US); Whitlock, Laura Lee, Highland Park Illinois 60045 (US)
(74) Representative: Schnappauf, Georg
(86) International application number: PCT/US2010/053166
(87) International publication number: WO 2011/049932

(56) References cited:
- EP-A1- 0 723 576
- WO-A1-01/19507
- WO-A1-91/13959
- WO-A1-92/06156
- WO-A1-92/06161
- WO-A1-95/06106
- WO-A1-98/28392
- WO-A1-2006/084190
- US-A- 5 244 652
- US-A- 5 616 781
- US-A- 6 087 320
- US-A1- 2007 010 680
- US-A1- 2009 200 511
- US-A1- 2009 200 511
- US-B1- 6 306 805
- J. G. OTTEN, C. L. NESTOR: "Anionic Hydretropes for Industrial and Institutional Rinse Aids", JAOCS, vol. 63, no. 8, August 1986 (1986-08), pages 1078-1079, XP002744267,

## Description

### BACKGROUND OF THE INVENTION

Development of personal care products, including, without limitation, liquid hand soaps, body washes, shampoos, 2-in-1 or 3-in-1 shampoos, bath washes, foaming hair conditioners, facial cleaners, among others, have been driven by the challenge of providing a combination of performance properties such as good foaming, good cleansing, good rinsing, enhanced mildness and improved skin feel. Often, the addition of a component to a cleansing composition formulation may enhance one property to the detriment of another desired property of the composition or further end product. Therefore, those in the art have been seeking new formulations to help achieve the balance of desirable performance properties. Recently, there has been a trend in personal care products to develop products that are mild and comprise ingredients that are naturally derived rather than synthetic.

Salts of alpha sulfonated fatty acid esters and salts of alpha sulfonated fatty acids have been used in detergents as primary surfactants. They have excellent foaming and cleansing properties in liquid dishwashing detergents and heavy duty liquids. US Patents 5,616,781, 5,637,758 and 5,945,394 provide descriptions of an overview of those hydrotropic surfactants in liquid dishwashing detergents and heavy duty liquids. However, due to the hydrotropic properties of salts of alpha sulfonated fatty acid esters and salts of alpha sulfonated fatty acid, products containing these materials usually have viscosities less than 1,000 centipoise (cps) at 25°C. In fact, sulfonated fatty acid salts have been used as viscosity reducing agents in liquid detergents or paste (US Patent 3,377,290). Building viscosity of personal care finished products based on salts of alpha sulfonated fatty acid esters and salts of alpha sulfonated fatty acids has been a challenge in the surfactant industry for many years.

In some cleansing applications, higher viscosity is required for the product's handling or ease of application. In addition, higher viscosity personal care products are more aesthetically appealing to consumers. For example, the viscosity for a shampoo or a body wash is typically higher than 2,000 cps at ambient temperature.

Polymeric thickeners have been used to build viscosity of compositions based on salts of alpha sulfonated fatty acid esters and salts of alpha sulfonated fatty acids. However, using polymeric thickeners can alter the product performance. It may make the product stringing, sticky, and slimy. In some cases, polymers had a negative impact on foaming and cleansing performance of formulated products. In addition, polymeric thickeners are more expensive than surfactants, and they do not have desirable cost/performance efficiency in cleansing products.

WO 95/06106 discloses a liquid or gel detergent composition comprising (a) a surfactant selected from anionic, nonionic and amphoteric surfactants, or mixtures thereof, (b) calcium ions, (c) a disulfonate surfactant and (d) water. WO 92/06161 describes a detergent composition comprising anionic sulfate or sulfonate surfactants, polyhydroxy fatty acid amides, and a critically selected suds enhancing agent. WO 91/13959 relates to a light-duty liquid dishwashing detergent composition comprising (a) an alkyl polysaccharide surfactant and (b) an alpha-sulfonated fatty acid alkyl ester surfactant in a weight ratio of (a)/(b) of from 50/50 to 95/5, and an optional suds booster. WO 98/28392 discloses a detergent composition comprising (a) an effective amount of alkanolamine, (b) a detersive effective amount of amphoteric surfactant, (c) a detersive effective amount of divalent ions, and (d) a chelant. WO 92/06156 describes a detergent composition comprising anionic sulfate or sulfonate surfactants, polyhydroxy fatty acid amides and magnesium ions. WO 2006/084190 discloses a liquid cleansing composition comprising a primary surfactant mixture of (i) sulfonated fatty acid/fatty acid ester surfactants combined with (ii) an alkyl sulfoacetate, an ethoxylated sulfoacetate or a mixture thereof in a particular ratio. WO 95/10585 relates to detergent compositions comprising alpha-sulfonated fatty acid methyl ester surfactants and anionic surfactants. US 6,306,805 relates to surfactant compositions comprising a mixture of at least one cationic surfactant, at least one anionic surfactant, preferably a sulfonated methyl ester and/or a sulfonated fatty acid, and at least one "bridging surfactant" selected from semi-polar nonionic, ethoxylated alkanolamide, and amphoteric/zwitterionic surfactants, and mixtures thereof.

### BRIEF SUMMARY OF THE INVENTION

The present technology relates to the discovery of surfactant thickeners that can be effectively used in combination with electrolytes to increase the viscosity of cleansing compositions comprising alpha sulfonated fatty acid esters and/or alpha sulfonated fatty acids and/or salts thereof. The present technology also relates to cleansing compositions that can be used as liquid cleansing consumer products. The use of the surfactant thickener of the present disclosure further improves foaming, cleansing and the perceptual skin feel properties of finished cleansing products comprising alpha sulfonated fatty acid esters, alpha sulfonated fatty acids, or salts thereof.

As one aspect of the present technology, a viscous liquid cleansing composition is provided. The cleansing composition consists of a) at least one hydrotropic surfactant selected from the group consisting of an alpha sulfonated fatty acid, an ester thereof, a salt of said acid or said ester, and a combination thereof having the structure of Formula 1: 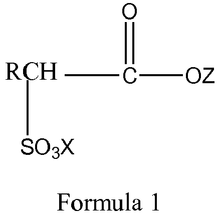 wherein R is a C₆-C₂₀ hydrocarbyl group; Z is straight or branched chain C₁-C₆ hydrocarbyl, or X; and X is H, Na, K, Ca, Mg, NH₄, monoethanolammonium, diethanolammonium, triethanolammonium or a mixture thereof, such as a salt of alpha sulfonated fatty acid ester or a salt of alpha sulfonated fatty acid, preferably a mixture of a mono-salt of alpha sulfonated fatty acid ester and a di-salt of alpha sulfonated fatty acid (preferably the alpha sulfonated fatty acid ester and/or the alpha sulfonated fatty acid has a selected distribution of alkyl chains, for example, a C₁₂-C₁₈ distribution); b) a mixture of two or more of alkyl betaines and/or alkyl sultaines having different alkyl chain lengths, wherein the alkyl betaines have the structure of Formula 2 and the alkyl sultaines have the structure of Formula 3: 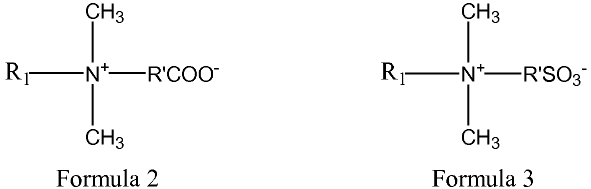 wherein R₁ is a C₈-C₂₂ hydrocarbyl group, R' is C₁-C₅ alkyl, hydroxyl alkyl, alkoxylated alkyl or a combination thereof; c) from 0% to 3% electrolyte; d) water present in an amount to balance the total composition to 100%; and e) optionally one or more other surfactants or additives; wherein the composition has a viscosity of at least 1,500 cps at 25°C as measured by a Brookfield RVT viscometer using spindle number 4 or number 5 at speed of 20 rpm, the other surfactants are selected from the group consisting of anionic surfactants, non-ionic surfactants, amphoteric surfactants, zwitterionic surfactants, semi-polar nonionic surfactants, cationic surfactants, and mixtures thereof, wherein the non-ionic surfactants are selected from the group consisting of alkyl glucosides, alkyl alcohols, alkyl alcohol ethoxylates, alkyl phenyl ethoxylates, propylene glycols, propylene glycol esters, ethylene glycol esters, ethoxylated glycol esters, polypropylene glycol esters, sucrose esters, sucrotriglycerides, alkyl fatty ester alkoxylates, alkyl glucamides, sorbitan esters, sucrose triglycerides, polyglycerol esters, fatty acid amides, ethoxylated fatty acid amides, alkyl lactyllactates and combinations thereof, and the additives are selected from the group consisting of emollients, skin conditioning agents, emulsifiers, suspending agents, fragrances, colors, herbal extracts, vitamins, builders, enzymes, pH adjusters, chelator, amino acids, sensorial modifiers, skin wrinkle reduction, ultra violet absorbing agents, exfoliating agents, preservatives, antibacterial agents, and polymeric additives, wherein the polymeric additives are selected from the group consisting of polyacrylic acids and the salts thereof, polyacrylates, polyacrylamides, copolymers of acrylate and acrylamide, copolymers of acrylate and hydroxyester acrylate, polyvinyl alcohols, polyethylene glycols, polyvinylacetates, polyvinyl pyrrolidones, hydroxylethyl cellulose, hydroxylmethyl cellulose, modified starches, modified xanthan pyrrogum, cationic cellulose, cationic starches, modified guar gum, copolymers of vinyl pyrrolidone and dimethylaminoethylmethacrylate, copolymers of vinyl pyrrolidone and vinyl acetate, copolymers of carboxylated vinyl acetate, polyethylene glycol, polyethylene glycol esters, derivatives thereof, and combinations thereof. In one embodiment of the viscous liquid cleansing composition of the present technology, in Formula 1 Z is -CH₃, ethyl or X. In one embodiment of the viscous liquid cleansing composition of the present technology, R is a distribution of alkyl chain lengths. In one embodiment of the viscous liquid cleansing composition of the present technology, the electrolyte is from one or more of the following salts: sodium chloride, potassium chloride, magnesium chloride, calcium chloride, ammonium chloride, sodium sulfate, potassium sulfate, magnesium sulfate, sodium citrate, sodium lactate, sodium glutamate, and mixtures thereof. In one embodiment of the viscous liquid cleansing composition of the present technology, the composition comprises at least one additional surfactant selected from the group consisting of anionic surfactants, non-ionic surfactants, zwitterionic surfactants, semi-polar nonionic surfactants, cationic surfactants, amphoteric surfactants, and mixtures thereof. In one embodiment of the viscous liquid cleansing composition of the present technology, the composition comprises one or more emollients, skin conditioning agents, pearlescent agents, emulsifiers, suspending agents, fragrances, colors, herbal extracts, vitamins, builders, chelator, amino acids, sensorial modifiers, skin wrinkle reduction, ultra violet absorbing agents, exfoliating agents, enzymes, pH adjusters, preservatives, antibacterial agents, or polymers. In one particular embodiment of the present technology, the viscous liquid cleansing composition consists of:
a) from 2% to 70%, more preferably from 3% to 50%, and most preferably from 5% to 30%, by actives weight of the hydrotropic surfactant;
b) from 1% to 50%, more preferably from 1% to 30%, and most preferably from 2% to 20%, by actives weight of the mixture of two or more alkyl betaines and/or alkyl sultaines;
c) from 0% to 3% by actives weight of an electrolyte; and
d) from 0% to 50% by actives weight of other surfactants and additives; and
e) water present in an amount to balance the total composition to 100%.
In the particular embodiment, the cleansing composition may comprise from 3% to 30% by actives weight of the hydrotropic surfactant. In the particular embodiment, the cleansing composition may comprise from 2% to 20% by actives weight of the mixture of two or more alkyl betaines and/or alkyl sultaines. Preferably the composition has an active concentration from 1.5% to 80%. In any embodiment of the viscous liquid cleansing composition of the present technology, the composition may be a body wash, hand wash, facial cleanser, shampoo, foaming hair conditioner, pumpable liquid hand cleanser, bath wash, liquid dish wash, or liquid detergent.

In various embodiments of the present technology, the hydrotropic surfactant is an alpha sulfonated fatty acid, an ester thereof, or a salt of such an acid or such an ester, having the structure of Formula 1: 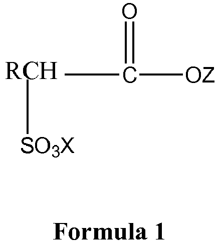 where **R** is a C₆-C₂₀ hydrocarbyl group, preferably an alkyl or other hydrocarbyl group, or a combination thereof, **Z** is -CH₃, ethyl or **X**, where **X** is H, Na, K, Ca, Mg, NH₄, monoethanolammonium, diethanolammonium, triethanolammonium or a mixture thereof. The hydrotropic surfactant can be a combination of such a sulfonated fatty acid, ester, or salt. **R** can represent a distribution of alkyl chain lengths.

The present compositions and formulations include an alkyl betaine having the structure of Formula 2 or an alkyl sultaine having the structure of Formula 3, or mixtures of said alkyl betaine and/or said alkyl sultaine: 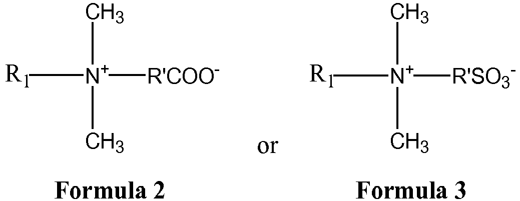 where R₁ is a C₈-C₂₂ hydrocarbyl group, preferably alkyl or a combination of alkyl and other hydrocarbyl groups, R' is C₁-C₅ alkyl, hydroxyl alkyl, alkoxylated alkyl or combination of thereof.

The present compositions and formulations preferably include an electrolyte from one or more of the following salts: sodium chloride, potassium chloride, magnesium chloride, calcium chloride, ammonium chloride, sodium sulfate, potassium sulfate, magnesium sulfate, sodium citrate, sodium lactate, sodium glutamate, and combinations thereof. The compositions and formulations can also include at least one other surfactant selected from the group consisting of anionic surfactants, non-ionic surfactants, amphoteric surfactants, zwitterionic surfactants, semi-polar nonionic surfactants, cationic surfactants, and mixtures thereof. The additives optionally included in the present compositions and formulations can be emollients, skin or hair conditioning agents, pearlescent agents, emulsifiers, suspending agents, fragrances, colors, herbal extracts, vitamins, builders, enzymes, pH adjusters, preservatives, antibacterial agents, or polymers.

### BRIEF DESCRIPTION OF THE DRAWINGS

Figure 1 illustrates the high viscosities obtained in various formulations comprising mixtures of alkyl dimethyl betaines having different alkyl chain lengths and salts of alpha sulfonated fatty acid ester or salts of alpha sulfonated fatty acid (Example 2 is not according to the present invention).
Figure 2 illustrates the improvement in foaming demonstrated by embodiments of the present disclosure. Examples 11-14 demonstrated higher foam volume compared to Example 2 (not according to the present invention) with or without the presence of oil.

### DETAILED DESCRIPTION OF THE INVENTION

The term "hydrotropic surfactant" refers to a compound that simultaneously behaves as (1) a hydrotrope, a compound with the ability to increase the solubilities of certain slightly watersoluble organic compounds and metal salts of organic compounds, and (2) a surfactant, a watersoluble compound that reduces the surface tension of liquids, or reduces interfacial tension between two liquids or a liquid and a solid. These hydrotropic surfactants also act as sequesterants for divalent metallic salts and solubilizers for metal salts of organic compounds.

The hydrotropic surfactant as specified in the claims can be an alpha sulfonated fatty acid, an ester of an alpha sulfonated fatty acid, a di-cation salt (di-salt) of an alpha sulfonated fatty acid, a mono-cation salt (mono-salt) of an alpha sulfonated ester of a fatty acid, or a blend of any of the foregoing acids, esters or salts. Preferably, the hydrotropic surfactant comprises a mono-cation salt (mono-salt) of an alpha sulfonated methyl ester of a fatty acid and a di-cation salt (di-salt) of an alpha sulfonated fatty acid, the ratio of mono-salt to di-salt being at least 1:1. Such mono-cation salts and di-cation salts can be included in an aqueous composition, such as the personal care compositions and formulations described herein. Since such salts may dissociate to some extent, a composition comprising such salts is understood to refer to a composition comprising the dissociated ions contributed by such salts unless otherwise indicated. Accordingly, the present disclosure includes any product resulting from the combination of a di-cation salt (di-salt) of an alpha sulfonated fatty acid, a mono-cation salt (mono-salt) of an alpha sulfonated ester of a fatty acid, or combinations thereof, in an aqueous solution with other elements set forth herein (such as an alkyl betaine or sultaine and/or an electrolyte).

The hydrotropic surfactant is present in the present compositions at concentrations of from 1% to 30% by active weight. Preferred compositions contain from 2.5 to 20% by active weight hydrotropic surfactant. The most preferred compositions contain from 3.5 to 15% by active weight hydrotropic surfactant.

The alpha sulfonated alkyl ester employed for making the present compositions may be pure alkyl ester or a blend of (1) a mono-salt of an alpha sulfonated alkyl ester of a fatty acid having from 8-20 carbon atoms where the alkyl portion forming the ester is straight or branched chain alkyl of 1-6 carbon atoms and (2) a di-salt of an alpha sulfonated fatty acid, the ratio of mono-salt to di-salt being at least 1:1. The alpha sulfonated alkyl esters used in the present technology can be prepared by sulfonating an alkyl ester of a fatty acid with a sulfonating agent such as SO₃. When prepared in this manner, the alpha sulfonated alkyl esters normally contain a minor amount, not exceeding 33% by weight, of the di-salt of the alpha sulfonated fatty acid which results from hydrolysis of the ester. Preferred alpha sulfonated alkyl esters contain less than 10% by weight of the di-salt of the corresponding alpha sulfonated fatty acid.

The alpha sulfonated alkyl esters, also known as alkyl ester sulfonate surfactants, include linear esters of C₈ to C₂₀ carboxylic acid (i.e., fatty acids) which are sulfonated with gaseous SO₃ according to the "The Journal of American Oil Chemists Society," 52 (1975), pp. 323-329. Suitable starting materials would include natural fatty substances as derived from coco, tallow, palm oil.

The alpha sulfonated fatty acid esters, alpha sulfonated fatty acids, and salts thereof, have the structure of Formula 1: 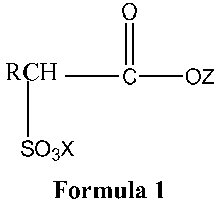 where **R** is a C₆-C₂₀ hydrocarbyl group, preferably an alkyl group or a combination of alkyl and other hydrocarbyl groups; **Z** is straight or branched chain C₁-C₆ hydrocarbyl, preferably an alkyl, or X, where X is H, Na, K, Ca, Mg, NH₄, monoethanolammonium, diethanolammonium, triethanolammonium or a mixture thereof. Exemplary products in this category include ALPHA-STEP® PC-48, ALPHA-STEP® MC-48, ALPHA-STEP® BSS-45, and ALPHA-STEP® P-65 from Stepan Company, Northfield, Illinois. **R** can be a distribution of alkyl chain lengths, for example one of the alkyl chain distributions set forth below with respect to alkyl betaines and alkyl sultaines.

The present compositions and formulations also include an alkyl betaine or an alkyl sultaine. The term "alkyl betaine" refers to compounds having the general structure of Formula 2, and the term "alkyl sultaine" refers to compounds having the general structure of Formula 3: 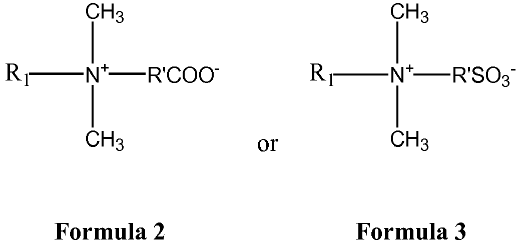 where R₁ is a C₈-C₂₂ hydrocarbyl group, preferably an alkyl group or a combination of alkyl and other hydrocarbyl groups; R' is C₁-C₅ alkyl, hydroxyl alkyl, alkoxylated alkyl, or a combination thereof. It follows from the foregoing Formula 2 that the term alkyl betaine does not include alkylamidopropyl betaines, and the term alkyl sultaine does not include alkylamidopropyl sultaines. The present technology is based in part on the surprising discovery that formulations comprising alkyl betaines and/or alkyl sultaines have better properties than comparable formulations comprising cocoamidopropyl betaine. This is surprising because, in many prior art cleansing compositions, cocoamidopropyl betaine is a preferred betaine.

The present compositions and formulations include a mixture of two or more of alkyl betaines and/or alkyl sultaines having different alkyl chain lengths. For example, two or more alkyl betaines and/or alkyl sultaines can be included so as to provide a selected distribution of alkyl chain lengths in the alkyl betaines and/or alkyl sultaines present in the composition or formulations. As discussed in more detail below, it has been surprisingly found that a mixture of alkyl betaines having different alkyl chain lengths can improve viscosity building and foaming properties. The mixture of alkyl betaines or alkyl sultaines can have a selected distribution of alkyl chain lengths, such as a C8-C22 distribution, a C8-C20 distribution, a C8-C18 distribution, a C8-C16 distribution, a C8-C14 distribution, a C8-C12 distribution, a C8-C10 distribution, a C10-C22 distribution, a C10-C20 distribution, a C10-C18 distribution, a C10-C16 distribution, a C10-C14 distribution, a C10-C12 distribution, a C12-C22 distribution, a C12-C20 distribution, a C12-C18 distribution, a C12-C16 distribution, a C12-C14 distribution, a C14-C22 distribution, a C14-C20 distribution, a C14-C18 distribution, a C14-C16 distribution, a C16-C22 distribution, a C16-C20 distribution, a C16-C18 distribution, a C18-C22 distribution, a C18-C20 distribution, or a C20-C22 distribution. Preferably a mixture of alkyl dimethyl betaines or alkyl dimethyl sultaines has a C8 to C22 distribution of alkyl chain lengths, more preferably a C12 to C18 distribution; most preferably a C12 to C16 distribution. Preferred alkyl chain ratios between C8, or C10, or C12, or C14, or any combination thereof to C16, or C18, or C20 or C22, or any combination thereof are from 5/95 to 95/5. The mixture of alkyl betaines can be, for example, a mixture of two or more of cetyl dimethyl betaine, coco dimethyl betaine, stearyl dimethyl betaine, behenyl betaine and lauryl dimethyl betaine.

Also described herein are processes for manufacturing liquid cleansing compositions and/or for manufacturing alkyl betaines or sultaines to be included in such liquid cleansing compositions. A process is disclosed for manufacturing a mixture comprising one or more alkyl betaines or alkyl sultaines having different alkyl chain lengths by combining a mixture of alkyl tertiary amines (for example, cetyl dimethyl amine and coco dimethyl amine) and reacting the alkyl tertiary amines with other reagents (for example, monochloroacetic acid). An advantage of this process is avoiding the high viscosity or gelling of an alkyl betaine or sultaine having an alkyl chain length greater than C₁₄. Another process is disclosed for making one or more alkyl betaines or alkyl sultaines by adding one or more alkyl tertiary amines to a diluent comprising water and a sulfonated fatty acid ester and/or an alpha sulfonated fatty acid, or a salt of either or both, and reacting the alkyl tertiary amine(s) with other reagents (for example a halocarboxylic acid). Either or both of the foregoing processes for manufacturing an alkyl betaine or alkyl sultaine or mixtures of betaines and/or sultaines can be incorporated in a process for manufacturing a cleansing composition. For example, a process for manufacturing a liquid cleansing composition can comprise preparing a mixture of alkyl dimethyl betaines by combining coco dimethyl amine and cetyl dimethyl amine in ALPHA-STEP® PC-48, which includes water and sulfonated fatty acids, esters thereof and salts thereof, and reacting the tertiary amines with monochloroacetic acid to form a four component system of cetyl dimethyl betaine, coco dimethyl betaine, sodium sulfonated methyl C₁₂-C₁₈ ester (mono) and disodium sulfonated C₁₂-C₁₈ fatty acid.

The present technology also provides concentrates for liquid cleansing compositions. Such concentrates can be diluted with water or another solvent and optionally combined with other surfactants, salts and/or additives to provide a liquid cleansing composition suitable for end use.

The present compositions and formulations can also comprise one or more electrolytes. The term "electrolyte" includes substances that will provide ionic conductivity in water or in the present compositions and formulations, or when in contact with them; such substances may either be solid or liquid. The term "salt" includes ionic compounds that provide one or more electrolytes when dissolved in water or when in contact with it. The salt(s) can be organic or inorganic. Preferred salts are sodium chloride, potassium chloride, magnesium chloride, calcium chloride, ammonium chloride, sodium sulfate, potassium sulfate, magnesium sulfate, sodium citrate, sodium lactate, sodium glutamate, and combinations thereof. Preferred electrolytes are those that result from the dissociation of the preferred salts. The salts are typically solids before addition or inclusion in the present liquid cleansing compositions, but then dissociate partially or completely so as to provide electrolytes in the liquid compositions.

The cleansing composition of the present technology may also contain other optional ingredients suitable for use, such as other surfactants and additives. The additional surfactants can be anionic, non-ionic, amphoteric, zwitterionic, semi-polar nonionic, cationic, and mixtures thereof.

Examples of anionic surfactants suitable for use with the present technology include, without limitation, sulfonated alkyl benzenes, sulfonated alpha olefins, paraffin sulfonated, alkyl sulfates, alkyl alkoxy sulfates, alkyl alkoxy carboxylates, alkyl phosphates, alkyl alkoxy phosphates, alkyl sulfonated and alkyl alkoxylated sulfates, fatty soaps, acyl lactylates, alkyl sulfoacetates, alkyl sulfosuccinates, alkyl alkoxy sulfosuccinates, acyl glutamates, alkyl sarcosinates, acyl methyl taurates, acyl isethionates, acyl amphoacetates, and combinations or mixtures thereof. Further examples are given in "Surface Active Agents and Detergents" (Vols. I and II by Schwartz, Perry and Berch). Further examples of anionic surfactants suitable for use with the present technology are also generally disclosed under the heading "Anionic Detergents" in U.S. Pat. No. 3,929,678, issued Dec. 30, 1975 to Laughlin, et al., at Column 23, line 58 through Column 29, line 23.

The nonionic surfactants suitable for use with the present technology are selected from the group consisting of alkyl glucosides, alkyl alcohols, alkyl alcohol ethoxylates, alkyl phenyl ethoxylates, propylene glycols, propylene glycol esters, ethylene glycol esters, ethoxylated glycol esters, polypropylene glycol esters, sucrose esters, sucrotriglycerides, alkyl fatty ester alkoxylates, alkyl glucamides, sorbitan esters, sucrose triglycerides, polyglycerol esters, fatty acid amides, ethoxylated fatty acid amides, alkyl lactyllactates and combinations thereof.

Examples of cationic surfactants suitable for use with the present technology include, without limitation, alkyl dimethylammonium halogenide, quaternized cellulose, quaternized guar gum, TEA esteramine esterquat, amidoquat, and stearylamidopropyl dimethyl amine quat, and combinations thereof. Further examples of cationic surfactants suitable for use with the present technology are also generally disclosed in US Pat. No. 4,228,044, to Cambre, issued Oct. 14, 1980.

Examples of zwitterionic surfactants suitable for use with the present technology include, without limitation, amine oxides, amidopropyl betaines, amidopropyl sultaines, amphoacetates, and propionates. Further examples of zwitterionic surfactants suitable for use with the present technology are also generally disclosed in US Pat. No. 3,929,678, to Laughlin et al., issued Dec. 30, 1975, at Column 19, line 38 through column 22, line 48.

Suitable polymeric additives and rheological modifiers for use with the present technology can be polymers or copolymers, and can be in anionic, nonionic, amphoteric or cationic forms. Suitable polymeric additives for use with the present technology are preferably water soluble or water dispersible. Polymeric additives suitable for use with the present technology are selected from the group consisting of polyacrylic acids and the salts thereof, polyacrylates, polyacrylamides, copolymers of acrylate and acrylamide, copolymers of acrylate and hydroxyester acrylate, polyvinyl alcohols, polyethylene glycols, polyvinylacetates, polyvinyl pyrrolidones, hydroxylethyl cellulose, hydroxylmethyl cellulose, modified starches, modified xanthan pyrrogum, cationic cellulose, cationic starches, modified guar gum, copolymers of vinyl pyrrolidone and dimethylaminoethylmethacrylate, copolymers of vinyl pyrrolidone and vinyl acetate, copolymers of carboxylated vinyl acetate, polyethylene glycol, polyethylene glycol esters, derivatives thereof, and combinations thereof.

Other optional ingredients suitable for use with the present technology are selected from the group consisting of emollients, skin conditioning agents, emulsifier/suspending agents, fragrances, colors, herbal extracts, vitamins, builders, enzymes, pH adjusters, chelator, amino acids, sensorial modifiers, skin wrinkle reduction, ultra violet absorbing agents, exfoliating agents, preservatives, and antibacterial agents. Some examples of emollients suitable for use with the present technology include, without limitation, vegetable oils, mineral oils, silicone oils, petrolatums, polyglycerol methyl esters, esters, glycerine and free fatty acid.

The cleansing compositions of the present technology have a viscosity at 25°C of at least 1,500 cps, alternatively at least 2,000 cps, alternatively at least 2,500 cps, alternatively at least 3,000 cps, alternatively at least 3,500 cps. The preferred viscosity range is up to 50,000 cps. The more preferred viscosity is between 2,500 and 20,000 cps. The most preferred viscosity range is between 3,500 and 15,000 cps.

The cleansing compositions of the present technology also demonstrated improvement in foaming and skin feel over existing cleaning compositions. These benefits will be demonstrated with examples provided.

Yet, it was surprisingly found that the alkyl betaines or sultaines with a selected distribution of alkyl chain lengths exhibited better viscosity building and foaming properties compared to a "narrow cut" alkyl betaines or sultaines. The "narrow cut" refers to a narrow alkyl chain distribution such as in lauryl betaine or stearyl betaine. The selected distribution of alkyl chain lengths can also improve the processability of long chain betaines or sultaines, such as cetyl betaine, stearyl betaine, behenyl betaine, cetyl sultaine, stearyl sultaine and behenyl sultaine. It was also found that alkyl betaines or sultaines with the selected alkyl chain distribution were more efficient in building the viscosity of compositions based on anionic surfactants compared to the commonly used cocoamidopropyl betaine. The synergy of the alkyl betaine distribution mixtures will be demonstrated in the examples provided.

Some suitable liquid cleansing compositions of the present technology that comprise hydrotropic surfactants include, for example, personal care skin cleansing products and hair care products. The personal care compositions can comprise hydrotropic surfactants of Formula 1 for example, in an amount from 0.1% to 99%, alternatively 0.5% to 99%, alternatively 1.0% to 99%, alternatively 1.0% to 80%, alternatively 2% to 70%, alternatively 2% to 60%, alternatively 3% to 50%, alternatively 5% to 30%, alternatively 5% to 20% by actives weight of the compositions. The present technology also relates to personal care or cleansing concentrates that include at least 20%, alternatively at least 30%, alternatively at least 40%, alternatively at least 50%, alternatively at least 60%, alternatively at least 70% of a hydrotropic surfactant, such percentages being by actives weight in the concentrate. The foregoing includes any range or percentage there between.

Some embodiments of the present technology provide a composition of a personal care product including 1% to 85% by actives weight of the composition of a mixture of two or more of alkyl betaines and/or alkyl sultaines having different alkyl chain lengths, preferably 1% to 50%, more preferably 1% to 30%, most preferably 2% to 20% by weight actives of said mixture. Alternatively, the mixture of two or more of alkyl betaines and/or alkyl sultaines having different alkyl chain lengths can be from 1% to 75%, from 1% to 60%, from 1% to 50%, from 1% to 40%, from 1% to 30%, from 2% to 20%, from 2% to 15%, from 3% to 10%, or from 3% to 5%. The present technology also relates to personal care or cleansing concentrates that include the mixture of two or more of alkyl betaines and/or alkyl sultaines having different alkyl chain lengths in an amount from 5% to 70%, alternatively from 5% to 60%, alternatively from 5% to 50%, alternatively from 5% to 40%, alternatively from 5% to 30%, alternatively from 5% to 20%, alternatively from 5% to 10%, alternatively from 10% to 60%, alternatively from 10% to 50%, alternatively from 10% to 40%, alternatively from 10% to 30%, alternatively from 10% to 20%, alternatively from 15% to 60%, from 20% to 40% by weight of the composition, alternatively from 1% to 10%, from 1% to 20%, alternatively between 5% and 30% by weight of the concentrate. The foregoing includes any percentage or range there between.

Some embodiments of the present technology provide a liquid cleansing composition comprising 0% to 3% by actives weight of the composition of at least one electrolyte, or a liquid cleansing composition prepared from components that include 0.5% to 2% by actives weight of the composition of at least one salt. In some embodiments of the present technology, compositions as described above further comprise an additional surfactant. The additional surfactant can be 0.1% to 85% by actives weight of the personal care composition, preferably 0.1% to 50% by actives weight of the personal care composition, alternatively from 0.1% to 30% by actives weight. Alternatively, the additional surfactant can be from 0.1% to 75%, from 0.1% to 60%, from 0.1% to 50%, from 0.1% to 40%, from 0.1% to 30%, from 0.1% to 20%, from 0.1% to 15%, from 0.1% to 10%, from 0.1% to 5%, alternatively from 1% to 75%, from 1% to 60%, from 1% to 50%, from 1% to 40%, from 1% to 30%, from 1% to 20%, from 1% to 15%, from 1% to 10%, from 1% to 5%, alternatively from 5% to 70%, alternatively from 5% to 60%, alternatively from 5% to 50%, alternatively from 5% to 40%, alternatively from 5% to 30%, alternatively from 5% to 20%, alternatively from 5% to 10%, alternatively from 10% to 60%, alternatively from 10% to 50%, alternatively from 10% to 40%, alternatively from 10% to 30%, alternatively from 10% to 20%, alternatively from 15% to 60%, from 20% to 40% by weight of the composition, alternatively from 1% to 10%, from 1% to 20%, alternatively between 5% and 30% by actives weight of the composition, and includes any percentage or range there between.

The liquid cleansing compositions described herein are preferably in the form of liquids in which water is the principal carrier/vehicle/diluent. The amount of water in a liquid cleansing composition is preferably from 3% to 99% by weight of the composition. A sufficient amount of water can be added to balance the total composition to 100%.

Again, as will be appreciated by at least those skilled in the art, a variety of carriers, vehicles, diluents, are suitable for use in the practice of the present disclosure in a non-exhaustive manner. Thus, it will also be appreciated that the terms carrier, vehicle, and diluent are to be considered non-exhaustive and interchangeable with respect to the present technology and in describing the various formulations, applications, compositions.

In some embodiments of the present disclosure, the compositions of Formula 1 can be included in personal care products or hair care products to help solubilize water insoluble ingredients, reduce viscosity and increase or reduce foaming capabilities. Personal care compositions of the present technology may be formulated to provide a desirable viscosity and foaming ability depending on the application. For example, pumpable or finger pump foamer hand cleansers may be desirable that have a viscosity which is pleasing to the feel but allows a proper quantity of the formulation to be readily delivered through an appropriately sized aperture of a hand pumped delivery apparatus.

In some embodiments of the present disclosure, the addition of a salt of alpha sulfonated fatty acid ester or a salt of alpha sulfonated fatty acid according to Formula 1 can be used to adjust the viscosity of the products to meet the desired use or the specifications of the regions or country in which the personal care composition is used. For example, formulations with a viscosity of from 1,000 cPs (i.e., centipoises) to 3,000 cPs are contemplated for some applications while viscosities from 2,000 cPs to 20,000 cPs as measured at 25°C using a Brookfield Viscometer model RVT, spindle #4 or #5, having a speed of 20 rpm are contemplated for other applications.

The formulations of the presently described technology may be used alone as a liquid cleansing composition, preferably as a body wash, hand wash, facial cleanser, shampoo, foaming hair conditioner. Alternatively, other optional ingredients may be added to make the compositions more preferable for a variety of different uses such as a pumpable liquid hand cleanser, 2-in-1 shampoo, gel body wash, bath wash, liquid dish wash, liquid detergent, among other end-products.

Optionally, the personal care product composition can include at least one additive. Suitable additives include, but are not limited to, for example, viscosity modifiers, electrolytes, thickeners, emollients, skin conditioning agents, emulsifier/suspending agents, fragrances, colors, herbal extracts, vitamins, builders, enzymes, pH adjusters, chelator, amino acids, sensorial modifiers, skin wrinkle reduction, ultra violet absorbing agents, exfoliating agent preservatives, antimicrobial agents (e.g., antibacterial agents, antiviral agents, antifungal agents, antiprotozoal agents, antihelmenthic agents, combinations thereof, among others), antidandruff agents and other ingredients commonly known in the art.

For example, additional thickeners may be added if necessary to achieve a desired viscosity for a particular cleansing composition. Such thickening agents may include, for example, monomeric thickening agents, such as esterquat, amidoquat, stearylamidopropyl dimethyl amine quat, or polymeric thickening agents such as cellulosic polymers, and acrylic polymers and copolymers. Alternatively, the cleansing products may be thickened by using polymeric additives that hydrate, swell or molecularly associate to provide body, such as, for example, hydroxypropyl guar gum. Other suitable thickening agents may include, without limitation, those listed in the Glossary and Chapters 3, 4, 12 and 13 of the Handbook of WaterSoluble Gums and Resins, Robert L. Davidson, McGraw-Hill Book Co., New York, NY 1980. Fatty acid soaps, builders, and additional surfactants may be added to aid in cleansing ability. Emollients (including, without limitation, vegetable oils, mineral oils, silicone oils, petrolatum, polyglycerol methyl esters, and esters), skin conditioning agents (such as glycerine and free fatty acid), vitamins and herbal extracts may be added to further improve conditioning performance. Fragrances, dyes, opacifying agents, and pearlescent agents may also be added to further enhance the appearance and olfactory property of the finished formulation.

Builders suitable for use in the practice of the present technology are, for example, those agents used in cleaning compositions whose major purpose is to counter the detrimental effects of polyvalent cations such as calcium and magnesium on detergency. In addition, builders serve to increase the detersive efficiency and effectiveness of surfactants and to supplement their beneficial effects on soil removal. Examples of builders suitable for use in the practice of the present technology include, but are not limited to sodium citrate, polycarboxylate, sodium carbonate, sodium aluminosilicate (e.g., Zeolite A, commercially available from PQ Corporation, Valley Forge, Pennsylvania.), among others. Additional builders suitable for use in the practice of the present technology are described in "Surfactants and Interfacial Phenomena", Third Edition, by Milton J. Rosen published by John Wiley & Sons, Inc. Hoboken: New Jersey (2004).

Preservatives for use in the formulations of the present technology are any suitable preservatives for personal care products and include, but are not limited to, acidics and phenolics, for example, benzoic acid and salts, sorbic acid and salts, propionic acid and salts, boric acid and salts, dehydroacetic acid, sulfurous and vanillic acids, Ottasept® (which is available from Ottawa Chemical Company (Toledo, OH)), Irgasan DP 300® (which is available from Geigy Chemical Corporation (Ardsley, NY)). Additional suitable preservatives for personal care products can be found in Preservatives for Cosmetics Manual, Second Edition, by David S. Steinbens, 2006.

Suitable antimicrobial agents for use in the practice of the present technology include, but are not limited to one or more antibacterial agents, antiviral agents, antiprotozoal agents, antihelminthic agents, antifungal agents, derivatives thereof, or combinations thereof. For example, suitable antimicrobial agents can be found in McCutcheons' 2009 Functional Materials of North American Edition, Volume 2, 2009, pages 239-246.

Other suitable antimicrobials include, but are not limited to, LDL antimicrobial components, triclosan, n-alkyl dimethyl benzyl ammonium chloride, n-alkyl dimethyl benzyl ammonium chloride, dialkyl dimethyl ammonium chloride, didecyl dimethyl ammonium chloride, dioctyl dimethyl ammonium chloride, phenolics, iodophors, pine oil, methyl salicylate, morpholine, silver, copper, bromine, and quaternary ammonium compounds, derivatives thereof, and combinations thereof including, but not limited to, the polyquaternium series as is used in hand soap formulations, and 3,4,4'-trichlorocarbanilide, as disclosed in US 6,605,579.

Additionally, silicone derivatives, for example, a dimethyl polysiloxane may be utilized to enhance skin feel and conditioning properties to hair. Furthermore, an antidandruff agent may be utilized to control dandruff on the scalp of a human subject.

The compositions and the methods of producing such compositions herein may be formulated and carried out such that they will have a pH of between 4.0 to 8.5, preferably between 5.0 to 7.0, alternatively between 5.0 to 6.5, alternatively between 5.0 to 6.0. Techniques for controlling pH at recommended usage levels include the use of buffers, alkali, acids, etc., and are well known to those skilled in the art. Optional pH adjusting agents can include, but are not limited to citric acid, succinic acid, phosphoric acid, sodium hydroxide, sodium carbonate.

### EXAMPLES

The following examples will further describe the present technology in detail. These examples are not meant to limit the scope of this invention, since variations will be apparent to those skilled in the art. Those examples which are not encompassed by the claims are given for comparative purposes only.

Performance Evaluation Table A provides trade names and description of various components used in exemplary compositions of the present technology.

**Table A**

| | |
|---|---|
| **Trade Names and Abbreviations** | |
| ALPHA-STEP® PC-48 | Sodium Sulfonated Methyl C₁₂-C₁₈ ester (mono) and Disodium Sulfonated C₁₂-C₁₈ fatty acid (di) with average mono- to di- ratio of approximately 7:1, available from Stepan Company, Northfield, Illinois. |
| ALPHA-STEP® MC-48 | |
| ALPHA-STEP® P-65 | Sodium Sulfonated Methyl C₁₄-C₁₈ ester (mono) and Disodium Sulfonated C₁₄-C₁₈ fatty acid (di) with average mono- to di- ratio of approximately 10:1, available from Stepan Company, Northfield, Illinois. |
| STEOL® CS-230 | Sodium Salt of C₁₂-C₁₄ Alkyl Ethoxy Sulfate with 2 moles Ethylene Oxide per mole of alcohol, available from Stepan Company, Northfield, Illinois. |
| AMPHOSOL® CDB Special | C₁₂-C₁₆ alkyl dimethyl betaine |
| AMPHOSOL® HCG | Cocoamidopropyl betaine, made from coco triglyceride, available from Stepan Company, Northfield, Illinois. |
| AMPHOSOL® HCA | Cocoamidopropyl betaine, made from coco methyl ester, available from Stepan Company, Northfield, Illinois. |
| AMPHOSOL® LB | Laurylamidopropyl betaine, available from Stepan Company, Northfield, Illinois. |
| AMPHOSOL® 2C | Disodium cocoamphodiacetate, available from Stepan Company, Northfield, Illinois. |
| AMPHOSOL® 1C | Sodium Cocoamphoacetate, available from Stepan Company, Northfield, Illinois. |
| STEPAN-MILD® L3 | Lauryl Lactyl Lactate, available from Stepan Company, Northfield, Illinois. |
| STEPAN-MILD® SL3 BA | Disodium Laureth Sulfosuccinate, available from Stepan Company, Northfield, Illinois. |
| BIOTERGE® AS-40 | Sodium C₁₄-C₁₆ olefin sulfonate, available from Stepan Company, Northfield, Illinois. |
| CEDAPAL® TD-403 | Sodium Trideceth Sulfate, available from Stepan Company, Northfield, Illinois. |
| STEPANOL® WA-EXTRA | Sodium lauryl sulfate, available from Stepan Company, Northfield, Illinois. |
| BIOSOFT® N25-7 | Alcohol ethoxylates based on a synthetic C₁₂-C₁₅ alcohol base with 7 moles of ethylene oxide, available from Stepan Company, Northfield, Illinois. |
| POLYSTEP® OPA | Sulfonated fatty acid, available from Stepan Company, Northfield, Illinois. |
| PEG 6000 DS | PEG-150 Distearate |
| AMMONYX® CO | Cetamine Oxide, available from Stepan Company, Northfield, Illinois. |
| Glucopon 625 UP | Alkylpolyglucosides, available from Cognis Corp, Cincinnati, Ohio |
| MAPROSYL® 30 | Sodium Lauroyl Sarcosinate, available from Stepan Company, Northfield, IL |
| STEPAN® SLL-FB | Sodium Lauroyl Lactylate, available from Stepan Company, Northfield, Illinois. |
| Hostapon CT | Sodium Cocoyl Methyl Taurate, available from Clariant, Charlotte, North Carolina. |
| Hostapon SCI-85C | Sodium cocoyl isethionate, available from Clariant, Charlotte, North Carolina. |
| Perlastan SC | Sodium cocoyl glutamate, available from Struktol Company, of America, Stow, Ohio |
| NINOL® COMF | Cocamide Monoethanol Amine (MEA), available from Stepan Company, Northfield, Illinois. |

Each of the exemplary compositions, as well as any compositions labeled "control composition," were prepared in de-ionized water. Materials used in all examples are expressed in percentage of an active material. The final pH of each composition was adjusted to between 5-6 using 25% solution of either sodium hydroxide or citric acid.

### Viscosity Determination Test Method:

The viscosity was measured with a Brookfield RVT viscometer using spindle number 4 or number 5 at a speed of either 10 or 20 rpm. The viscosity of some formulations was also determined with a Rheologist AR2000 rheometer (from TA instruments) using 4cm cone-plate geometry at 25°C and 1 1/s (reciprocal second) shear rate.

### Cylinder Inversion Foam Test Method:

1. Prepare a 0.2% active sample solution in the 25 °C tap water.
2. Add 100.0g of the 0.2% sample solution to a 500 ml graduated cylinder. Keep the foam to a minimum.
3. Add 2.0g of castor oil to the graduated cylinder and stopper the cylinder.
4. Place the graduated cylinder in the mechanical shake foam machine. Invert cylinder 10 times.
5. Allow the foam to settle for 15 seconds. Record an initial reading of total foam height. Record foam height again after 5 minutes.

### Human Panel Test Method:

Skin feel evaluation for the examples was carried out using an in-vivo human expert panel test. Three to twelve trained panelists with different skin types (dry, normal, and moist) were chosen for each test. The skin type of the panelist was determined using a moisture meter available from Nova Technology Corporation, Portsmouth, New Hampshire, referred to herein as a Nova meter. A reading by the Nova meter of less than 100 represents dry skin, 110-130, normal skin and 130 or above, moist skin. The panelists were asked to each assess the performance of each composition tested on a scale of 1 to 5, with 1 being the worst and 5 being the best. The individual assessments of the panelists were then averaged and recorded for each composition tested.

In the test that used a control composition, the results of which are shown in Table 6 provided below, the average score of the control composition was subtracted from the score of each test composition to arrive at a relative comparison number. The score of the control sample for each property is zero, and the scores for the test compositions are either positive, negative, or zero. A positive number thus indicates that the tested formulation performed better than the control. A negative number indicates that the formulation performed worse than control, and zero indicates equal performance between sample and control.

During testing, the panelists were asked to wash their hands in accordance with the procedure described below, and to assess the following characteristics during the washing and drying procedure: flash foam, foam feel, foam volume and tackiness. Skin softness, skin dryness and skin tightness were evaluated after skin is completely dry. To identify tackiness during drying, the panelists were instructed that some products might impart a sticky/tacky feel on the skin during the transition from a wet to a dry stage. Tackiness can be assessed by touching the fingers of the same hand together or by force required to separate fingers. To identify skin tightness when dry, the panelists were instructed that some products may leave the skin feeling tight or stretched after the skin is completely dry. The panelists were instructed that this property should not be evaluated until the panelist is sure that the hands are completely dry. Similarly, skin dryness was evaluated once the hands were completely dry. To identify skin softness, the panelists were instructed to characterize how soft and smooth the skin feels to the touch. A product can often leave the skin feeling dry, but smooth. The positive extreme would be a smooth velvety feel, and the opposite would be a rough feeling skin with some grittiness. All samples were coded in order to obtain a fair and unbiased comparison.

### For each composition:

1. Panelists were asked to pre-wash their hands with the composition, to remove residue from the skin and establish the baseline before evaluation.
2. Tests were conducted using luke-warm (35°C (95°F) and 40.6°C (105°F)) tap water.
3. Using a syringe, 1 ml of the 15% liquid cleansing product was dispensed to the panelist's wet palm, followed by 1 ml of water.
4. The panelists were asked to wash their hands by gently rubbing them together for 30 seconds followed by rinsing under running water for 15 seconds.
5. Foam generated during hand wash process was collected and transferred into a graduated beaker. Foam volume was measured and recorded.
6. Skin feel evaluation was done at ambient temperature (25°C).

### Salon Half-head Test Method:

1. Comb dry hair and divide into 2 sections (half head). Thoroughly wet hair.
2. Using a disposable syringe, apply 4 ml of each shampoo, control on one side, experimental on the other side.
3. Wash each side using eight circular motions to work up foam.
4. Evaluate the control and experimental shampoo for foam volume, density, stability, and rinsability.
5. Rinse hair with tap water for 10 seconds. Repeat procedures 1-4 using 2 ml of each shampoo. Then rinse for 10 seconds.
6. Evaluate the control and experimental shampoo for foam volume, density, stability, and rinsability.
7. Using a plastic comb, after the second shampooing, evaluate the hair for detangling and wet comb ability properties.
8. Blow-dry the hair and evaluate for dry comb ability, static, body, and shine.

The performance of each composition was tested on a scale of 0 to 3 compared to the control, with 0 being equal performance, 1 being slightly better, 2 being noticeably better, and 3 being obviously better. The individual assessments of each panelist was then averaged and recorded for each composition tested.

Examples 1-10 (not according to the present invention) in Table 1 illustrate high viscosity cleansing compositions of the present disclosure comprising a salt of alpha sulfonated fatty acid ester and a salt of alpha sulfonated fatty acid in combination with alkyl dimethyl betaines. Controls 1 and 2 are formulations with alkyl amidopropyl betaine in combination with a salt of alpha sulfonated fatty acid ester and a salt of alpha sulfonated fatty acid. Example 7 and Control 1 are derived from the same coco (C8-18) alkyl chain source; Example 8 and Control 2 are derived from the same lauryl (C12) alkyl chain source. However, the formulations differ in that Examples 7 and 8 contain an alkyl betaine, while Controls 1 and 2 contain an alkyl amidopropyl betaine. Under the same test conditions, Example 7 and Example 8 have viscosity of more than 3,000 cps, while the Control formulations 1 and 2 have viscosity of 200 cps or less. These results suggest that the alkyl dimethyl betaine without the amido linkage is the key molecular structure to build the viscosity of the compositions based on salts of alpha sulfonated fatty acid esters and salts of alpha sulfonated fatty acid.

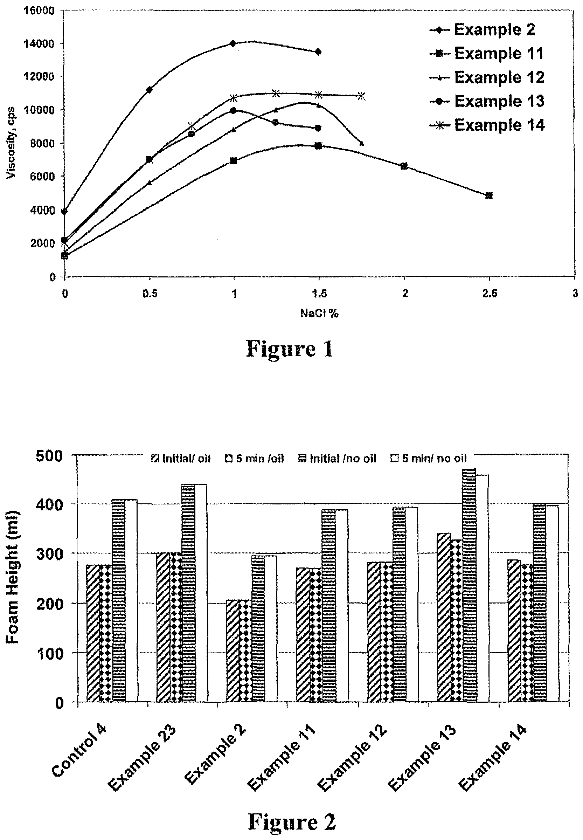

Examples 11-19 in Table 2 demonstrate the mixtures of alkyl dimethyl betaines having different alkyl chain lengths as thickeners for the compositions with a salt of alpha sulfonated fatty acid ester and a salt of alpha sulfonated fatty acid. These mixtures of alkyl dimethyl betaine can efficiently build the viscosity of the formulations based on a salt of alpha sulfonated fatty acid ester or a salt of alpha sulfonated fatty acid. Comparing Example 19 to the Control formulation 3, it was further proved that the alkyl dimethyl structure is the key to build the viscosity of compositions based on a salt of alpha sulfonated fatty acid ester and a salt of alpha sulfonated fatty acid. By adding appropriate concentration of salt, the viscosity in the excess of 10,000 cps can be achieved for some of the example formulations. The viscosity salt response of Example 2 and Examples 11-14 is illustrated in Figure 1. Examples 17 and 18 are examples of personal care concentrates.

In addition, it was surprisingly found that a selected distribution of alkyl chain lengths in a mixture of alkyl dimethyl betaines can improve the foaming performance of a cleansing composition comprising salts of alpha sulfonated fatty acid ester and salts of alpha sulfonated fatty acid when compared to a "pure cut" alky dimethyl betaine, such as cetyl (C₁₆) betaine. The improvement in foaming is shown in Figure 2. Examples 11-14 demonstrate higher foam volume compared to Example 2 with or without the presence of castor oil. Examples 11-14 also have equal foaming performance compared to the Control 4 formulation, which is the most commonly used cleansing composition in personal care applications today.

The viscous liquid cleansing compositions comprising a salt of alpha sulfonated fatty acid ester and/or a salt of alpha sulfonated fatty acid can also be combined with other surfactants and additives. Examples 20-40 (Examples 24 to 30 are not according to the present invention) in Tables 3 and 4 demonstrate the concept of combining the present disclosure with other surfactants and additives.

Examples 41 and 42 (not according to the present invention) are described in Table 5 and they demonstrate that alkyl dimethyl sultaines provide an advantage similar to alkyl dimethyl betaines in building viscosity of compositions based on a salt of alpha sulfonated fatty acid ester and/or a salt of alpha sulfonated fatty acid. However, Controls 5 and 6 demonstrate that alkyl amidopropyl sultaines did not have the same viscosity-building advantage as alkyl dimethyl sultaine. These results once again show that alkyl betaines or alkyl sultaines are significantly and surprisingly different compared to alkylamidopropyl betaines or alkylamidopropyl sultaines in terms of viscosity building of cleansing compositions based on hydrotropic surfactants.

The thickening effect of alkyl betaines or alkyl sultaines has been described in great detail in the above examples. In addition, it was surprisingly found that the viscous liquid cleansing compositions comprising a salt of alpha sulfonated fatty acid ester and a salt of alpha sulfonated fatty acid with the selected alkyl chain distribution of alkyl betaine improved the foaming performance and skin after-feel, based on the in-vivo expert panel hand washing test. The performance of Examples 13 and 23 using in-vivo expert panel was compared to the Control 4. The results of this study are shown in Table 6.

The scores given in Table 6 represent the average number using twelve panelists. The results demonstrate that the panelists preferred the experimental formulations to the control in both foaming and skin after-feel. The flash foam and foam volume for experimental formulations of the present technology performed significantly better compared to the control. The foam volume for Examples 13 and 23 is 55% and 65% higher respectively compared to the Control 4.

Example 23 of the present technology was tested as a shampoo using half-head salon test method against Control 4 and a leading commercial shampoo. The average results from three panelists are shown in Table 7. The results in Table 7 show that the composition of the present technology (Example 23) had better foaming than both Control 4 and the leading commercial shampoo.

**Table 1. Examples of Thickened Cleansing Compositions Based on Salts of Alpha Sulfonated Fatty Acid Esters and Alpha Sulfonated Fatty Acids (ALPHA-STEP® PC-48) and Alkyl Dimethyl Betaines**

| | Example 1* | Example 2* | Example 3* | Example 4* | Example 5* | Example 6* | Example 7* | Example 8* | Example 9* | Example 10* | Control 1 | Control 2 |
|---|---|---|---|---|---|---|---|---|---|---|---|---|
| | % Act. | % Act. | % Act. | % Act. | % Act. | % Act. | % Act. | % Act. | % Act. | % Act. | % Act. | % Act. |
| ALPHA-STEP® PC-48 | 11.25 | 10 | 9 | 7.5 | 5 | 8 | 10 | 10 | 2 | 1 | 10 | 10 |
| Cetyl Dimethyl Betaine | 3.75 | 5 | 6 | 7.5 | 10 | 4 | | | 3 | 1 | | |
| Coco Dimethyl Betaine | | | | | | | 5 | | | | | |
| Lauryl Dimethyl Betaine | | | | | | | | 5 | | | | |
| AMPHOSOL® HCG | | | | | | | | | | | 5 | |
| AMPHOSOL® LB | | | | | | | | | | | | 5 |
| NaCl | 2.5 | 0 | 0 | 0 | 0 | 1 | 2 | 2 | 1 | 1 | 2 | 2 |
| Total Active | 15 | 15 | 15 | 15 | 15 | 12 | 15 | 15 | 5 | 2 | 15 | 15 |
| Viscosity (cps) @25°C | 2,500 | 3,900 | 21,200 | 16,500 | 4,900 | 5,800 | 3,000 | 3,300 | 3,200 | 1,300 | <100 | 200 |

| | | | | | | | | | | | | |
|---|---|---|---|---|---|---|---|---|---|---|---|---|
| *: not according to the present invention | | | | | | | | | | | | |

**Table 2. Examples of Thickened Cleansing Compositions Based on Salts of Alpha Sulfonated Fatty Acid Esters and Alpha Sulfonated Fatty Acids (ALPHA-STEP® PC-48) and Mixtures of Alkyl Dimethyl Betaines**

| | **Example 11** | **Example 12** | **Example 13** | **Example 14** | **Example 15** | **Example 16** | **Example 17** | **Example 18** | **Example 19** | **Control 3** | Control 4 |
|---|---|---|---|---|---|---|---|---|---|---|---|
| | % Act. | % Act. | % Act. | % Act. | % Act. | % Act. | % Act. | % Act. | % Act. | % Act. | % Act. |
| ALPHA-STEP® PC-48 | 10 | 10 | 10 | 10 | 10 | 10 | 34.9 | 24.4 | 16.7 | 16.7 | |
| Cetyl Dimethyl Betaine | 2.5 | 3 | 3.5 | 4 | 1.28 | | 12.2 | 8.5 | 5.8 | | |
| Coco Dimethyl Betaine | 2.5 | 2 | 1.5 | 1 | 0.57 | 1.5 | 5.3 | 3.7 | 2.5 | | |
| Stearyl Dimethyl Betaine | | | | | 3.15 | 3.5 | | | | | |
| STEOL® CS-230 | | | | | | | | | | | 12 |
| AMPHOSOL® HCG | | | | | | | | | | 8.3 | 3 |
| NaCl | 2 | 1 | 1 | 1 | 1.5 | 2 | 0 | 0 | 0 | 0 | 1.5 |
| Total active | 15 | 15 | 15 | 15 | 15 | 15 | 52.4 | 36.6 | 25 | 25 | 15 |
| Viscosity (cps) @25°C | 5,600 | 8,800 | 9,900 | 10,700 | 9,700 | 7,400 | 3,700 | 6,000 | 20,800 | 100 | 9,000 |

**Table 3. Examples of Thickened Cleansing Compositions Based on Salts of Alpha Sulfonated Fatty Acid Esters and Alpha Sulfonated Fatty Acids (ALPHA-STEP® PC-48), Alkyl Dimethyl Betaines and Other Additives**

| | **Example 20** | **Example 21** | **Example 22** | **Example 23** | **Example 24*** | **Example 25*** | **Example 26*** | **Example 27*** | **Example 28*** | **Example 29*** | **Example 30*** |
|---|---|---|---|---|---|---|---|---|---|---|---|
| | % Act. | % Act. | % Act. | % Act. | % Act. | % Act. | % Act. | % Act. | % Act. | % Act. | % Act. |
| ALPHA-STEP® PC-48 | 10 | 10 | 10 | 10 | 10 | 10 | 10 | 10 | 10 | 10 | 10 |
| Cetyl Dimethyl Betaine | 3.5 | 3.15 | 3.15 | 3.15 | 5 | 3.5 | 3.5 | 3.5 | 3.5 | 3.5 | 2.5 |
| Coco Dimethyl Betaine | 1.5 | 1.35 | 1.35 | 1.35 | | | | | | | |
| STEPAN-MILD® L3 | 0.5 | | | | | | 1.5 | | | | |
| Sodium Cocoyl Isethionate | | 0.5 | | | | | | | | | |
| Glucopon® 625 UP | | | 0.5 | | | | | | | | |
| STEPAN® SLL-FB | | | | 0.5 | | | | | | | |
| Polyquaterium 7 | | | | | 0.3 | | | | | | |
| AMPHOSOL® 1C | | | | | | | | 1.5 | | | |
| AMPHOSOL® 2C | | | | | | | | | 1.5 | | |
| AMPHOSOL® SL3-BA | | | | | | 1.5 | | | | 1.5 | |
| AMPHOSOL® HCG | | | | | | | | | | | 2.5 |
| NaCl | 0 | 2 | 2 | 2 | 0.25 | 2.5 | 1 | 2.5 | 1 | 2.5 | 2 |
| Total Active | 15.5 | 15 | 15 | 15 | 15.3 | 15 | 15 | 15 | 15 | 15 | 15 |
| Viscosity (cps) @25°C | 6,600 | 4,400 | 5,800 | 5,500 | 7,800 | 3,900 | 4,300 | 7,400 | 3,800 | 1,700 | 2,200 |

| | | | | | | | | | | | |
|---|---|---|---|---|---|---|---|---|---|---|---|
| *: not according to the present invention | | | | | | | | | | | |

**Table 4. Examples of Thickened Cleansing Compositions Based on Salts of Alpha Sulfonated Fatty Acid Esters and Alpha Sulfonated Fatty Acids (ALPHA-STEP® PC-48), Alkyl Dimethyl Betaines, and Other Additives**

| | Example 31 | Example 32 | Example 33 | Example 34 | Example 35 | Example 36 | Example 37 | Example 38 | Example 39 | Example 40 |
|---|---|---|---|---|---|---|---|---|---|---|
| | % Act. | % Act. | % Act. | % Act. | % Act. | % Act. | % Act. | % Act. | % Act. | % Act. |
| ALPHA-STEP® PC-48 | 10 | 10 | 10 | 8 | 8 | 9 | 9 | 9 | 10 | 10 |
| Cetyl Dimethyl Betaine | 3.5 | 3.5 | 3 | 2.8 | 2.1 | 3.15 | 3.15 | 3.15 | 3.15 | 3.15 |
| Coco Dimethyl Betaine | 1.5 | 1.5 | 2 | 1.2 | 0.9 | 1.35 | 1.35 | 1.35 | 1.35 | 1.35 |
| Lauryl Alcohol | | | 0.5 | | | | | | | |
| CEDAPAL® TD-403 | | | | 3 | | | | | | |
| STEPANOL® WA-100 | | | | | 4 | | | | | |
| BIO-TERGE® AS-40 | | | | | | 1.5 | | | | |
| BIOSOFT® N-25 | | | | | | | 1.5 | | | |
| POLYSTEP® OPA | | | | | | | | 1 | | |
| STEPAN® PEG 6000 DS | | | | | | | | 0.5 | | |
| AMMONYX® CO | | | | | | | | | 0.5 | |
| NINOL® COMF | | | | | | | | | | 0.5 |
| NaSO₄ | 2 | | | | | | | | | |
| MgSO₄ | | 1.5 | | | | | | | | |
| NaCl | | | | 2.5 | 2.5 | 1.5 | 2.5 | 1.5 | 1.5 | 1.5 |
| Total Active | 15 | 15 | 15.5 | 15 | 15 | 15 | 15 | 15 | 15 | 15 |
| Viscosity (cps) @25°C | 3,400 | 5,400 | 8,700 | 2,700 | 1,500 | 4,200 | 2,000 | 2,700 | 4,800 | 3,600 |

**Table 5. Examples of Thickened Cleansing Compositions Based on Salts of Alpha Sulfonated Fatty Acid Esters and Alpha Sulfonated Fatty Acids (ALPHA-STEP® PC-48) and Alkyl Dimethyl Sultaines**

| | Example 41* | Example 42* | Control 5 | Control 6 |
|---|---|---|---|---|
| | % Act. | % Act. | % Act. | % Act. |
| ALPHA-STEP® PC-48 | 10 | 7.5 | 10 | 10 |
| Lauryl Dimethyl Sultaine | 5 | 7.5 | | |
| Cocoamidopropyl Sultaine | | | 5 | |
| Cetylamidopropyl Sultaine | | | | 5 |
| NaCl | 2 | 0 | 2 | 2 |
| Total Active % | 15 | 15 | 15 | 15 |
| Viscosity (cps) @25°C | 3,000 | 13,300 | <100 | <100 |

| | | | | |
|---|---|---|---|---|
| *: not according to the present invention | | | | |

**Table 6. In-Vivo Hand Washing Performance Results (12 Panelists)**

| *Formulation* | Flash Foam | Foam Volume (ml) | Foam Feel | Overall Impression Foam | Tackiness During Drying | Skin Tightness | Skin Dryness | Skin Softness | Overall Impression on Skin Feel |
|---|---|---|---|---|---|---|---|---|---|
| Control 4 | 0 | 70 | 0 | 0 | 0 | 0 | 0 | 0 | 0 |
| Example 23 | 0.75 | 116 | 0.17 | 0.75 | 0.33 | 0.25 | 0.17 | 0.25 | 0.42 |
| Example 13 | 0.58 | 110 | 0.17 | 0.83 | 0.25 | 0.17 | 0.33 | 0.25 | 0.33 |

**Table 7. In-Vivo Salon Half Head Salon Test Results (3 Panelists)**

| | Control 4 | Example 23 | Leading Commercial Sulfate Free Shampoo | Example 23 |
|---|---|---|---|---|
| First Application | 0 | 0 | 0 | 0 |
| Flash Foam | 0 | 0 | 0 | 0 |
| Volume | 0.3 | 1 | 0 | 1 |
| Stability | 0 | 0 | 0 | 0 |
| Density | 0 | 0 | 0 | 0 |
| Rinsibility | 0 | 0 | 0 | 0 |
| Second Application | 0 | 0 | 0 | 0 |
| Flash Foam | 0 | 0 | 0 | 0 |
| Volume | 0.3 | 1.3 | 0 | 2.3 |
| Stability | 0 | 0 | 0 | 0 |
| Density | 0 | 0 | 0 | 0 |
| Rinsibility | 0 | 0 | 0 | 0 |
| After Shampoo Condition | 0 | 0 | 0 | 0 |
| Detangling | 0 | 0 | 0 | 0 |
| Wet Comb-ability | 0 | 0 | 0 | 0 |
| Dry Comb-ability | 0 | 0 | 0 | 0 |
| Absence of Static | 0 | 0 | 0 | 0 |
| Body and Shine | 0 | 0 | 0 | 0 |

Yet another advantage of the present technology is the ease of manufacturing of alkyl betaines containing higher concentrations of cetyl (C₁₆) and stearyl (C₁₈) chain lengths.

A "pure cut" of cetyl betaine or stearyl betaine can be difficult to handle due to very high viscosity and its propensity for stringing. It was surprisingly found that a selected distribution of alkyl chain lengths in an alkyl betaine mixture could significantly improve the processing and handling of these products, due to a viscosity decrease. The mixture of alkyl betaine is preferred and can be preferentially made directly from a mixed alkyl tertiary amine feed to avoid the high viscosity (gelling) of cetyl betaine or stearyl betaine. Examples 44 to 46 in Table 8 demonstrate the benefit of the mixture of alkyl betaine with wide carbon distribution compared to "pure cut" cetyl betaine, Example 43 (not according to the present invention). The preferred alkyl chain distribution between C₈, or C₁₀, or C₁₂, or C₁₄, or the combination thereof to C₁₆, or C₁₈, or C₂₀, C₂₂ or the combination thereof is from 1/10 to 10/1.

The mixture of alkyl betaines or sultaines have improved foaming compared to the betaine or sultaine with single alkyl chain length or "pure cut" alkyl chain distribution of greater than C₁₄. The mixture of alkyl betaines or sultaines also have improved thickening properties compared to the betaine or sultaine with single alkyl chain length or "pure cut" alkyl chain distribution of less than or equal to C₁₄.

The mixture of alkyl betaine with a selected alkyl chain length distribution works as a very efficient thickener over the traditional alkyl amidopropyl betaine used in liquid cleansing systems. The efficiency of the mixture of alkyl betaine as a thickening agent is demonstrated by Examples 47-58 in Table 9. These examples can be compared in the following pairs: Example 47 vs. Example 48 (not according to the present invention); Example 49 vs. Example 50 (not according to the present invention); Example 51 vs. Example 52 (not according to the present invention); Example 53 vs. Example 54 (not according to the present invention); Example 55 vs. Example 56 (not according to the present invention); and Example 57 vs. Example 58 (not according to the present invention). At the same use concentration, the alkyl dimethyl betaine mixture is significantly more efficient than the alkyl amidopropyl betaine in thickening surfactant solutions.

Examples 59 and 60 illustrate liquid cleansing formulations containing surfactants and other additives such as skin and hair conditioning agents, foam boosters, humectants, polymers, colors, fragrance, pearlescent agent, pH adjuster, preservatives and other desired additives or functional materials. These formulated examples demonstrated desired viscosity without additional salt.

Examples 61, 62 and 63 (not according to the present invention) illustrate liquid cleansing formulations containing salts of alpha sulfonated fatty esters and alpha sulfonated fatty acids having different distributions of alkyl chains, along with an alkyl betaine. These formulated examples provide another demonstration that the present disclosure provides thickened cleansing compositions having excellent foaming properties.

**Table 8. Handling Comparison of Mixtures of Alkyl Dimethyl Betaine to Cetyl Betaine with Narrow Carbon Distribution**

| | Example 43* | Example 44 | Example 45 | Example 46 |
|---|---|---|---|---|
| | % Act. | % Act. | % Act. | % Act. |
| Cetyl Dimethyl Betaine | 29.2 | 22.4 | 23.0 | 25.6 |
| Coco Dimethyl Betaine | | 9.6 | 5.8 | 11.4 |
| Stearyl Dimethyl Betaine | | | | 62.9 |
| Total Active | 29.2 | 32 | 28.8 | 25.3 |
| Viscosity (cps) @25°C | 31,600 | 200 | 200 | 100 |
| Appearance | Very viscous and stringy | Clear flowable liquid | Clear flowable liquid | Low viscosity slurry |

| | | | | |
|---|---|---|---|---|
| *: not according to the present invention | | | | |

**Table 9. Comparison of Alkyl Dimethyl Betaine to Alkyl Amidopropyl Betaine as Thickeners for Different Anionic Surfactants**

| | Example 47 % Act. | Example 48* % Act. | Example 49 % Act. | Example 50* % Act. | Example 51 % Act. | Example 52* % Act. | Example 53 % Act. | Example 54* % Act. | Example 55 % Act. | Example 56* % Act. | Example 57 % Act. | Example 58* % Act. |
|---|---|---|---|---|---|---|---|---|---|---|---|---|
| STEOL® CS-230 | 8.8 | 8.8 | | | | | | | | | | |
| STEPANOL® WA-EXTRA | | | 7.2 | 7.2 | | | | | | | | |
| BIOTERGE® AS-40 | | | | | 8.8 | 8.8 | | | | | | |
| Hostapon CT | | | | | | | 10 | 10 | | | | |
| Maprosyl 30B | | | | | | | | | 9 | 9 | | |
| Perlastan SC | | | | | | | | | | | 10 | 10 |
| Cetyl Dimethyl Betaine | 2.2 | | 1.3 | | 2.2 | | 3.5 | | 4.2 | | 3.5 | |
| Coco Dimethyl Betaine | 1.0 | | 0.5 | | 1.0 | | 1.5 | | 1.8 | | 1.5 | |
| AMPHOSOL® HCA | | | | | | | | 5 | | 6 | | 5 |
| AMPHOSOL® HCG | | 3.2 | | 1.8 | | 3.2 | | | | | | |
| NaCl | 1.5 | 1.5 | 2 | 2 | 2 | 2 | 1.5 | 1.5 | 0.5 | 0.5 | 1 | 1 |
| Total active | 12 | 12 | 9 | 9 | 12 | 12 | 15 | 15 | 15 | 15 | 15 | 15 |
| Viscosity (cps) @25°C | 4,200 | 410 | 7,580 | 1,070 | 3,510 | 1 | 6,110 | 270 | 4,780 | 120 | 3,820 | 60 |

| | | | | | | | | | | | | |
|---|---|---|---|---|---|---|---|---|---|---|---|---|
| *: not according to the present invention | | | | | | | | | | | | |

**Table 10. Liquid Cleansing Examples with Additives**

| Ingredient | Example 59 % Act. | Example 60 % Act. |
|---|---|---|
| ALPHA-STEP® PC-48 | 10.00 | 6.00 |
| AMPHOSOL® CDB Special | 5.00 | 4.40 |
| STEPAN-MILD® L3 | 0.50 | |
| STEPAN® SLL-FB | 0.50 | |
| Hydrocoll EN-55 (Arch Chemical, Hydrolyzed Collagen) | 0.25 | |
| Flax Extract 130 (Natunola, LINIUM USITATISSIUM Extract) | 0.25 | |
| Mackernium 007 (The McINTYRE Group, Polyquaternium-7) | 0.25 | 0.25 |
| Panthenol (50%) (DSM, Panthenol) | 0.25 | |
| Glycol Stearate (Hallstar, EGDS) | 0.25 | |
| Glycerine | | 1.5 |
| Fragrance | 0.10 | 0.1 |
| Dye (Color) | q.s. | q.s. |
| Kathon CG | 0.05 | 0.05 |
| Citric Acid (25%) | q.s. | q.s. |
| Water | q.s. to 100 | q.s. to 100 |
| pH | 5.56 | 5.52 |
| Viscosity (cps) | 5,800 | 8,310 |

**Table 11. Liquid Cleansing Compositions Having Alpha Sulfonated Fatty Acid Esters and Alpha Sulfonated Fatty Acids Of Different Alkyl Chain Lengths**

| | Example 61* % Act. | Example 62* % Act. | Example 63* % Act. |
|---|---|---|---|
| ALPHA-STEP P-65 | 3.75 | 3.0 | 3.0 |
| ALPHA-STEP PC-48 | 0 | 3.0 | 1.5 |
| Coco Dimethyl Betaine | 3.75 | 3.0 | 3.0 |
| Citric Acid (25%) | q.s. | q.s. | q.s. |
| Sodium Chloride | 0 | 1.5 | 0.5 |
| Water | q.s. to 100 | q.s. to 100 | q.s. to 100 |
| Total Active | 7.5 | 9.0 | 7.5 |
| Appearance | Clear viscous gel | Clear viscous liquid | Clear viscous liquid |
| pH | 5.30 | 5.46 | 5.35 |
| Viscosity (cps) | gel | 11,400 | 13,750 |
| Foam Volume without Castor Oil (ml) | 330 | 410 | 375 |
| Foam Volume with 2% Castor Oil (ml) | 240 | 335 | 305 |

| | | | |
|---|---|---|---|
| *: not according to the present invention | | | |

## Claims

1. A viscous liquid cleansing composition consisting of:
a) at least one hydrotropic surfactant selected from the group consisting of an alpha sulfonated fatty acid, an ester thereof, a salt of said acid or said ester, and a combination thereof having the structure of Formula 1: 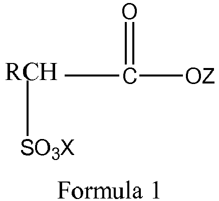 wherein R is a C₆-C₂₀ hydrocarbyl group; Z is straight or branched chain C₁-C₆ hydrocarbyl, or X; and X is H, Na, K, Ca, Mg, NH₄, monoethanolammonium, diethanolammonium, triethanolammonium or a mixture thereof;
b) a mixture of two or more of alkyl betaines and/or alkyl sultaines having different alkyl chain lengths, wherein the alkyl betaines have the structure of Formula 2 and the alkyl sultaines have the structure of Formula 3: 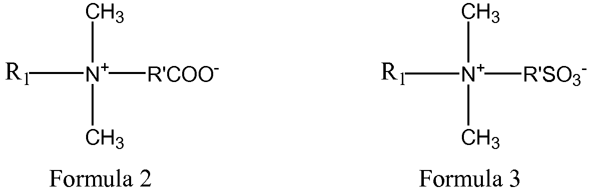 wherein R₁ is a C₈-C₂₂ hydrocarbyl group, R' is C₁-C₅ alkyl, hydroxyl alkyl, alkoxylated alkyl or a combination thereof;
c) from 0% to 3% by actives weight of electrolyte;
d) water; and
e) optionally one or more other surfactants or additives;
wherein the composition has a viscosity of at least 1,500 cps at 25°C as measured by a Brookfield RVT viscometer using spindle number 4 or number 5 at speed of 20 rpm,
the other surfactants are selected from the group consisting of anionic surfactants, non-ionic surfactants, amphoteric surfactants, zwitterionic surfactants, semi-polar nonionic surfactants, cationic surfactants, and mixtures thereof, wherein the non-ionic surfactants are selected from the group consisting of alkyl glucosides, alkyl alcohols, alkyl alcohol ethoxylates, alkyl phenyl ethoxylates, propylene glycols, propylene glycol esters, ethylene glycol esters, ethoxylated glycol esters, polypropylene glycol esters, sucrose esters, sucrotriglycerides, alkyl fatty ester alkoxylates, alkyl glucamides, sorbitan esters, sucrose triglycerides, polyglycerol esters, fatty acid amides, ethoxylated fatty acid amides, alkyl lactyllactates and combinations thereof, and the additives are selected from the group consisting of emollients, skin conditioning agents, emulsifiers, suspending agents, fragrances, colors, herbal extracts, vitamins, builders, enzymes, pH adjusters, chelator, amino acids, sensorial modifiers, skin wrinkle reduction, ultra violet absorbing agents, exfoliating agents, preservatives, antibacterial agents, and polymeric additives, wherein the polymeric additives are selected from the group consisting of polyacrylic acids and the salts thereof, polyacrylates, polyacrylamides, copolymers of acrylate and acrylamide, copolymers of acrylate and hydroxyester acrylate, polyvinyl alcohols, polyethylene glycols, polyvinylacetates, polyvinyl pyrrolidones, hydroxylethyl cellulose, hydroxylmethyl cellulose, modified starches, modified xanthan pyrrogum, cationic cellulose, cationic starches, modified guar gum, copolymers of vinyl pyrrolidone and dimethylaminoethylmethacrylate, copolymers of vinyl pyrrolidone and vinyl acetate, copolymers of carboxylated vinyl acetate, polyethylene glycol, polyethylene glycol esters, derivatives thereof, and combinations thereof.

2. The cleansing composition of claim 1, wherein in Formula 1 Z is -CH₃, ethyl or X.

3. The cleansing composition of claim 1, wherein R is a distribution of alkyl chain lengths.

4. The cleansing composition of claim 1, wherein the electrolyte is from one or more of the following salts: sodium chloride, potassium chloride, magnesium chloride, calcium chloride, ammonium chloride, sodium sulfate, potassium sulfate, magnesium sulfate, sodium citrate, sodium lactate, sodium glutamate, and mixtures thereof.

5. The cleansing composition of claim 1, wherein the composition comprises at least one additional surfactant selected from the group consisting of anionic surfactants, non-ionic surfactants, zwitterionic surfactants, semi-polar nonionic surfactants, cationic surfactants, amphoteric surfactants, and mixtures thereof.

6. The cleansing composition of claim 1, wherein the composition comprises one or more emollients, skin conditioning agents, pearlescent agents, emulsifiers, suspending agents, fragrances, colors, herbal extracts, vitamins, builders, chelator, amino acids, sensorial modifiers, skin wrinkle reduction, ultra violet absorbing agents, exfoliating agents, enzymes, pH adjusters, preservatives, antibacterial agents, or polymers.

7. The cleansing composition of claim 1 consisting of:
a) from 2% to 70% by actives weight of the hydrotropic surfactant;
b) from 1% to 50% by actives weight of the mixture of two or more alkyl betaines and/or alkyl sultaines;
c) from 0% to 3% by actives weight of an electrolyte;
d) from 0% to 50% by actives weight of other surfactants and additives; and
e) water present in an amount to balance the total composition to 100%.

8. The cleansing composition of claim 7, comprising from 3% to 30% by actives weight of the hydrotropic surfactant.

9. The cleansing composition of claim 7, comprising from 2% to 20% by actives weight of the mixture of two or more alkyl betaines and/or alkyl sultaines.

10. The cleansing composition of any one of claims 1 to 9, wherein the composition is a body wash, hand wash, facial cleanser, shampoo, foaming hair conditioner, pumpable liquid hand cleanser, bath wash, liquid dish wash, or liquid detergent.

## Patentansprüche

1. Viskose, flüssige Reinigungszusammensetzung, bestehend aus:
a) mindestens einem hydrotropen Tensid, ausgewählt aus der Gruppe, bestehend aus einer alpha-sulfonierten Fettsäure, einem Ester davon, einem Salz der Säure oder des Esters und einer Kombination davon mit der Struktur von Formel 1: 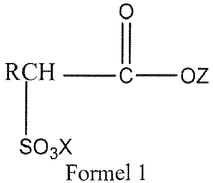 worin R eine C₆-C₂₀-Hydrocarbylgruppe ist; Z eine gerad- oder verzweigtkettige C₁-C₆-Hydrocarbylgruppe oder X ist; und X H, Na, K, Ca, Mg, NH₄, eine Monoethanolammonium-, Diethanolammonium-, Triethanolammoniumgruppe oder ein Gemisch davon ist;
b) einem Gemisch von zwei oder mehr Alkylbetainen und/oder Alkylsultainen mit unterschiedlichen Alkylkettenlängen, wobei die Alkylbetaine die Struktur von Formel 2 aufweisen und die Alkylsultaine die Struktur von Formel 3 aufweisen: 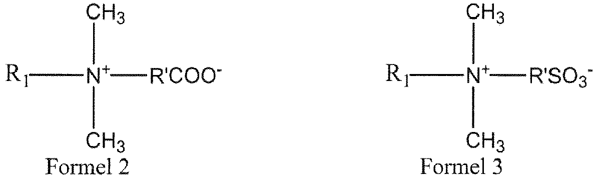 worin R₁ eine C₈-C₂₂-Hydrocarbylgruppe ist, R' eine C₁-C₅-Alkyl-, Hydroxylalkyl-, alkoxylierte Alkylgruppe oder eine Kombination davon ist;
c) 0% bis 3% nach Gewicht der Wirkstoffe an Elektrolyt;
d) Wasser; und
e) gegebenenfalls einem oder mehreren Tensiden oder Additiven;
wobei die Zusammensetzung eine Viskosität von mindestens 1.500 cps bei 25°C, wie mittels eines Brookfield RVT-Viskosimeters unter Verwendung von Spindel Nummer 4 oder Nummer 5 bei einer Geschwindigkeit von 20 UpM gemessen, aufweist,
wobei die anderen Tenside aus der Gruppe ausgewählt sind, die aus anionischen Tensiden, nicht-ionischen Tensiden, amphoteren Tensiden, zwitterionischen Tensiden, semipolaren nicht-ionischen Tensiden, kationischen Tensiden und Gemischen davon besteht, wobei die nicht-ionischen Tenside aus der Gruppe ausgewählt sind, die aus Alkylglucosiden, Alkylalkoholen,
Alkylalkoholethoxylaten, Alkylphenylethoxylaten, Propylenglykolen, Propylenglykolestern, Ethylenglykolestern, ethoxylierten Glykolestern, Polypropylenglykolestern, Sucroseestern, Sucrotriglyceriden, Alkylfettesteralkoxylaten, Alkylglucamiden, Sorbitanestem, Sucrosetriglyceriden, Polyglycerinestern, Fettsäureamiden, ethoxylierten Fettsäureamiden, Alkyllactyllactaten und Kombinationen davon besteht, und
die Additive aus der Gruppe ausgewählt sind, die aus Weichmachern, Hautkonditionierungsmitteln, Emulgatoren, Suspendiermittteln, Duftstoffen, Farbstoffen, Pflanzenextrakten, Vitaminen, Gerüststoffen, Enzymen, pH-Einstellmitteln, Chelator, Aminosäuren, Sinnesmodifizierungsmitteln, Hautfaltenreduktion, UV-Absorptionsmitteln, Peeling-Mitteln, Konservierungsmitteln, antibakteriellen Mitteln und polymeren Additiven besteht, wobei die polymeren Additive aus der Gruppe ausgewählt sind, die aus Polyacrylsäuren und den Salzen davon, Polyacrylaten, Polyacrylamiden, Copolymeren von Acrylat und Acrylamid, Copolymeren von Acrylat und Hydroxyesteracrylat, Polyvinylalkoholen, Polyethylenglykolen, Polyvinylacetaten, Polyvinylpyrrolidonen, Hydroxylethylcellulose, Hydroxylmethylcellulose, modifizierten Stärken, modifiziertem Xanthan-Pyrrogummi, kationischer Cellulose, kationischen Stärken, modifiziertem Guaran, Copolymeren von Vinylpyrrolidon und Dimethylaminoethylmethacrylat, Copolymeren von Vinylpyrrolidon und Vinylacetat, Copolymeren von carboxyliertem Vinylacetat, Polyethylenglykol, Polyethylenglykolestern, Derivaten davon und Kombinationen davon besteht.

2. Reinigungszusammensetzung nach Anspruch 1, wobei in Formel 1 Z -CH₃, eine Ethylgruppe oder X ist.

3. Reinigungszusammensetzung nach Anspruch 1, wobei R eine Verteilung von Alkylkettenlängen ist.

4. Reinigungszusammensetzung nach Anspruch 1, wobei der Elektrolyt einer oder mehr aus den nachstehenden Salzen ist: Natriumchlorid, Kaliumchlorid, Magnesiumchlorid, Calciumchlorid, Ammoniumchlorid, Natriumsulfat, Kaliumsulfat, Magnesiumsulfat, Natriumcitrat, Natriumlactat, Natriumglutamat und Gemische davon.

5. Reinigungszusammensetzung nach Anspruch 1, wobei die Zusammensetzung mindestens ein zusätzliches Tensid umfasst, das aus der Gruppe ausgewählt ist, die aus anionischen Tensiden, nicht-ionischen Tensiden, zwitterionischen Tensiden, semipolaren nicht-ionischen Tensiden, kationischen Tensiden, amphoteren Tensiden und Gemischen davon besteht.

6. Reinigungszusammensetzung nach Anspruch 1, wobei die Zusammensetzung mindestens eines oder mehrere aus Weichmachern, Hautkonditionierungsmitteln, Perlglanzmitteln, Emulgatoren, Suspendiermittteln, Duftstoffen, Farbstoffen, Pflanzenextrakten, Vitaminen, Gerüststoffen, Chelator, Aminosäuren, Sinnesmodifizierungsmitteln, Hautfaltenreduktion, UV-Absorptionsmitteln, Peeling-Mitteln, Enzymen, pH-Einstellmitteln, Konservierungsmitteln, antibakteriellen Mitteln oder Polymeren umfasst.

7. Reinigungszusammensetzung nach Anspruch 1, bestehend aus:
a) 2% bis 70% nach Gewicht der Wirkstoffe des hydrotropen Tensids;
b) 1% bis 50% nach Gewicht der Wirkstoffe des Gemisches von zwei oder mehr Alkylbetainen und/oder Alkylsultainen;
c) 0% bis 3% nach Gewicht der Wirkstoffe eines Elektrolyten;
d) 0% bis 50% nach Gewicht der Wirkstoffe an anderen Tensiden und Additiven; und
e) Wasser, das in einer Menge vorhanden ist, um die Gesamtzusammensetzung auf 100% auszugleichen.

8. Reinigungszusammensetzung nach Anspruch 7, die 3% bis 30% nach Gewicht der Wirkstoffe des hydrotropen Tensids umfasst.

9. Reinigungszusammensetzung nach Anspruch 7, die 2% bis 20% nach Gewicht der Wirkstoffe des Gemisches von zwei oder mehr Alkylbetainen und/oder Alkylsultainen umfasst.

10. Reinigungszusammensetzung nach einem jeglichen der Ansprüche 1 bis 9, wobei die Zusammensetzung ein Körperwaschmittel, Handwaschmittel, Gesichtsreinigungsmittel, Shampoo, schäumender Haarkonditionierer, pumpbares, flüssiges Handreinigungsmittel, Badewaschmittel, flüssiges Spülmittel oder flüssiges Detergens ist.

## Revendications

1. Composition nettoyante liquide visqueuse consistant en :
a) au moins un tensioactif hydrotrope choisi dans l'ensemble constitué par un acide gras alpha-sulfoné, un ester de celui-ci, un sel dudit acide ou dudit ester, et une combinaison de ceux-ci, ayant une structure de formule 1 : 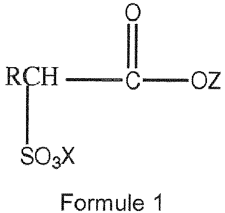 dans laquelle R est un groupe hydrocarbyle en C₆ à C₂₀ ; Z est un hydrocarbyle en C₁ à C₆ à chaîne droite ou ramifiée, ou X ; et X est H, Na, K, Ca, Mg, NH₄, le monoéthanolammonium, le diéthanolammonium, le triéthanolammonium ou un mélange de ceux-ci ;
b) un mélange de deux ou plus de deux alkylbétaïnes et/ou alkylsultaïnes ayant des longueurs de chaîne alkyle différentes, dans lequel les alkylbétaïnes ont une structure de formule 2 et les alkylsultaïnes ont une structure de formule 3 : 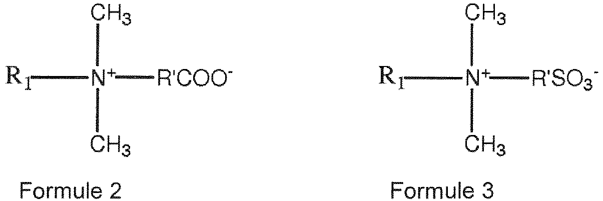 formules dans lesquelles R₁ est un groupe hydrocarbyle en C₈ à C₂₂, R' est un alkyle en C₁ à C₅, un hydroxyalkyle, un alkyle alcoxylé ou une combinaison de ceux-ci ;
c) de 0 % à 3 %, en poids des agents actifs, d'un électrolyte ;
d) de l'eau ; et
e) éventuellement un ou plusieurs tensioactifs ou additifs ;
laquelle composition a une viscosité d'au moins 1500 cps à 25°C, telle que mesurée au moyen d'un viscosimètre Brookfield RVT utilisant une broche numéro 4 ou numéro 5 à une vitesse de 20 t/min,
dans laquelle les autres tensioactifs sont choisis dans l'ensemble constitué par les tensioactifs anioniques, les tensioactifs non-ioniques, les tensioactifs amphotères, les tensioactifs zwittérioniques, les tensioactifs non-ioniques semi-polaires, les tensioactifs
cationiques, et leurs mélanges, dans laquelle les tensioactifs non-ioniques sont choisis dans l'ensemble constitué par les alkylglucosides, les alcools alkyliques, les alcools alkyliques éthoxylés, les éthoxylates d'alkylphényle, les propylèneglycols, les esters de propylèneglycol, les esters d'éthylèneglycol, les esters de glycol éthoxylés, les esters de polypropylèneglycol, les esters de saccharose, les triglycérides de sucre, les esters alkyliques gras alcoxylés, les alkylglucamides, les esters de sorbitan, les triglycérides de saccharose, les esters de polyglycérol, les amides d'acides gras, les amides d'acides gras éthoxylés, les lactyl-lactates d'alkyle et leurs combinaisons, et
les additifs sont choisis dans l'ensemble constitué par les émollients, les agents de conditionnement de la peau, les émulsionnants, les agents de mise en suspension, les parfums, les colorants, les extraits de plantes, les vitamines, les adjuvants, les enzymes, les agents d'ajustement du pH, les chélatants, les acides aminés, les modificateurs organoleptiques, les agents réduisant les rides, les agents absorbant les ultraviolets, les exfoliants, les conservateurs, les agents antibactériens, et les additifs polymères, lesquels additifs polymères sont choisis dans l'ensemble constitué par les poly(acides acryliques) et leurs sels, les polyacrylates, les polyacrylamides, les copolymères d'acrylate et d'acrylamide, les copolymères d'acrylate et d'hydroxyester-acrylate, les poly(alcools vinyliques), les polyéthylèneglycols, les poly(acétates de vinyle), les polyvinylpyrrolidones, l'hydroxyéthylcellulose, l'hydroxyméthylcellulose, les amidons modifiés, la pyrrogomme xanthane modifiée, la cellulose cationique, les amidons cationiques, la gomme de guar modifiée, les copolymères de vinylpyrrolidone et de méthacrylate de diméthylaminoéthyle, les copolymères de vinylpyrrolidone et d'acétate de vinyle, les copolymères d'acétate de vinyle carboxylé, le polyéthylèneglycol, les esters de polyéthylèneglycol, leurs dérivés, et leurs combinaisons.

2. Composition nettoyante selon la revendication 1, dans laquelle, dans la formule 1, Z est -CH₃, éthyle ou X.

3. Composition nettoyante selon la revendication 1, dans laquelle R est une distribution de longueurs de chaîne alkyle.

4. Composition nettoyante selon la revendication 1, dans laquelle l'électrolyte est un ou plusieurs des sels suivants : chlorure de sodium, chlorure de potassium, chlorure de magnésium, chlorure de calcium, chlorure d'ammonium, sulfate de sodium, sulfate de potassium, sulfate de magnésium, citrate de sodium, lactate de sodium, glutamate de sodium, et leurs mélanges.

5. Composition nettoyante selon la revendication 1, laquelle composition comprend au moins un tensioactif additionnel choisi dans l'ensemble constitué par les tensioactifs anioniques, les tensioactifs non-ioniques, les tensioactifs zwittérioniques, les tensioactifs non-ioniques semi-polaires, les tensioactifs cationiques, les tensioactifs amphotères, et leurs mélanges.

6. Composition nettoyante selon la revendication 1, laquelle composition comprend un ou plusieurs émollients, agents de conditionnement de la peau, agents nacrés, émulsionnants, agents de mise en suspension, parfums, colorants, extraits de plantes, vitamines, adjuvants, chélatants, acides aminés, modificateurs organoleptiques, agents réduisant les rides, agents absorbant les ultraviolets, exfoliants, enzymes, agents d'ajustement du pH, conservateurs, agents antibactériens, ou polymères.

7. Composition nettoyante selon la revendication 1, consistant en :
a) 2 % à 70 %, en poids des agents actifs, du tensioactif hydrotrope ;
b) 1 % à 50 %, en poids des agents actifs, du mélange de deux ou plus de deux alkylbétaïnes et/ou alkylsultaïnes ;
c) 0 % à 3 %, en poids des agents actifs, d'un électrolyte ;
d) 0 % à 50 %, en poids des agents actifs, d'autres tensioactifs et additifs ; et
e) de l'eau présente en une quantité pour porter la composition totale à 100 %.

8. Composition nettoyante selon la revendication 7, comprenant 3 % à 30 %, en poids des agents actifs, du tensioactif hydrotrope.

9. Composition nettoyante selon la revendication 7, comprenant 2 % à 20 %, en poids des agents actifs, du mélange de deux ou plus de deux alkylbétaïnes et/ou alkylsultaïnes.

10. Composition nettoyante selon l'une quelconque des revendications 1 à 9, laquelle composition est un nettoyant pour le corps, un nettoyant pour les mains, un nettoyant pour le visage, un shampooing, un conditionneur capillaire moussant, un nettoyant pour les mains liquide pouvant être pompé, un nettoyant pour le bain, un nettoyant liquide pour la vaisselle, ou un détergent liquide.
